(19) Europäisches Patentamt

European Patent Office

Office européen des brevets

(11) **EP 0 551 182 B1**

(12) **EUROPEAN PATENT SPECIFICATION**

(45) Date of publication and mention
of the grant of the patent:
**12.07.2000 Bulletin 2000/28**

(51) Int. Cl.[7]: **A61K 31/70**

(21) Application number: **93300052.3**

(22) Date of filing: **06.01.1993**

(54) **Method of treating hyperproliferative vascular disease using rapamycin, eventually in combination with mycophenolic acid**

Verfahren zur Behandlung von hyperproliferaktiven Gefässerkrankungen unter Verwendung von Rapamycin, eventuell in Kombination mit Mykophenolsäure

Méthode pour traiter des maladies vasculaires prolifératives en utilisant la rapamycine, éventuellement en combinaison avec l'aide mycophénolique

(84) Designated Contracting States:
**AT BE CH DE DK ES FR GB GR IE IT LI LU NL PT SE**

(30) Priority: **09.01.1992 US 819314**

(43) Date of publication of application:
**14.07.1993 Bulletin 1993/28**

(73) Proprietor:
**AMERICAN HOME PRODUCTS CORPORATION
Madison, New Jersey 07940-0874 (US)**

(72) Inventors:
• **Morris, Randall Ellis
Santa Clara, California (US)**
• **Gregory, Clare Robert
San Mateo, California (US)**

(74) Representative:
**Wileman, David Francis, Dr. et al
c/o Wyeth Laboratories
Huntercombe Lane South
Taplow Maidenhead Berkshire SL6 OPH (GB)**

(56) References cited:
**US-A- 5 078 999**

• **TRANSPLANTATION PROCEEDINGS vol. 23, no. 6, December 1991, pages 2833 - 2836 Y. AKSELBAND 'RAPAMYCIN INHIBITS SPONTANEOUS AND FIBROBLAST GROWTH FACTOR BETA-STIMULATED PROLIFERATION OF ENDOTHELIAL CELLS AND FIBROBLASTS'**
• **THE LANCET vol. 338, no. 8778, 23 November 1991, pages 1297 - 1298 B.M. MEISER ET AL. 'EFFECTS OF CYCLOSPORIN, FK506, AND RAPAMYCIN ON GRAFT-VESSEL DISEASE'**
• **CYTOKINE vol. 3, no. 5, September 1991, page 472 J. WOO ET AL. 'INFLUENCE OF IMMUNOSUPPRESSIVE MACROLIDES AND MYCOPHENOLIC ACID ON OKT3- AND IL-2-INDUCED HUMAN LYMPHOCYTE PROLIFERATION AND IL-2R EXPRESSION'**
• **FASEB J. vol. 6, no. 4, 1992, page A940 C. GREGORY ET AL. 'USE OF ANTIPROLIFERATIVE AGENTS FOR THE TREATMENT OF OCCLUSIVE VASCULAR DISEASE'**
• **PROC. NATL. ACAD. SCI. USA vol. 85, April 1988, pages 2303 - 2306 L. JONASSON ET AL. 'CYCLOSPORIN A INHIBITS SMOOTH MUSCLE PROLIFERATION IN THE VASCULAR RESPONSE TO INJURY'**

- **TRANSPLANTATION PROCEEDINGS vol. 23, no. 1, February 1991, pages 507 - 508 S.M. STEPKOWSKI ET AL. 'PROLONGATION BY RAPAMYCIN OF HEART, KIDNEY, PANCREAS, AND SMALL BOWEL ALLOGRAFT SURVIVAL IN RATS'**
- **TRANSPLANTATION vol. 50, no. 4, October 1990, pages 554 - 558 D.V. CRAMER ET AL. 'CARDIAC TRANSPLANTATION IN THE RAT'**
- **IMMUNOLOGY vol. 72, no. 4, April 1991, pages 544 - 549 J.E. KAY ET AL. 'INHIBITION OF T AND B LYMPHOCYTE PROLIFERATION BY RAPAMYCIN'**
- **SCAND. J. IMMUNOL. vol. 33, no. 2, February 1991, pages 161 - 173 E.M. EUGUI ET AL 'LYMPHOCYTE-SELECTIVE CYTOSTATIC AND IMMUNOSUPPRESSIVE EFFECTS OF MYCOPHENOLIC ACID IN VITRO: ROLE OF DEOXYGUANOSINE NUCLEOTIDE DEPLETION'**

# EP 0 551 182 B1

## Description

BACKGROUND OF THE INVENTION

Many individuals suffer from heart disease caused by a partial blockage of the blood vessels that supply the heart with nutrients. More severe blockage of blood vessels in such individuals often leads to hypertension, ischemic injury, stroke, or myocardial infarction. Typically vascular occlusion is preceded by vascular stenosis resulting from intimal smooth muscle cell hyperplasia. The underlying cause of the intimal smooth muscle cell hyperplasia is vascular smooth muscle injury and disruption of the integrity of the endothelial lining. The overall disease process can be termed a hyperproliferative vascular disease because of the etiology of the disease process. Intimal thickening following arterial injury can be divided into three sequential steps: 1 ) initiation of smooth muscle cell proliferation following vascular injury, 2) smooth muscle cell migration to the intima, and 3) further proliferation of smooth muscle cells in the intima with deposition of matrix. Investigations of the pathogenesis of intimal thickening have shown that, following arterial injury, platelets, endothelial cells, macrophages and smooth muscle cells release paracrine and autocrine growth factors (such as platelet derived growth factor, epidermal growth factor, insulin-like growth factor, and transforming growth factor) and cytokines that result in the smooth muscle cell proliferation and migration. T-cells and macrophages also migrate into the neointima. [Haudenschild, C., Lab. Invest. 41: 407 (1979); Clowes, A., Circ. Res. 56: 139 (1985); Clowes, A, J, Cardiovas. Pharm. 14 (Suppl. 6): S12 (1989); Manderson, J., Arterio. 9: 289 (1989); Forrester, J., J. Am. Coll. Cardiol. 17: 758 (1991)]. This cascade of events is not limited to arterial injury, but also occurs following injury to veins and arterioles.

Vascular injury causing intimal thickening can be broadly categorized as being either biologically or mechanically induced. Artherosclerosis is one of the most commonly occurring forms of biologically mediated vascular injury leading to stenosis. The migration and proliferation of vascular smooth muscle plays a crucial role in the pathogenisis of artherosclerosis. Artherosclerotic lesions include massive accumulation of lipid laden "foam cells" derived from monocyte/macrophage and smooth muscle cells. Formation of "foam cell" regions is associated with a breech of endothelial integrity and basal lamina destruction. Triggered by these events, restenosis is produced by a rapid and selective proliferation of vascular smooth muscle cells with increased new basal lamina (extracellular matrix) formation and results in eventual blocking of arterial pathways. [Davies, P.F., Artherosclerosis Lab. Invest. 55: 5 (1986)].

Mechanical injuries leading to intimal thickening result following balloon angioplasty, vascular surgery, transplantation surgery, and other similar invasive processes that disrupt vascular integrity. Intimal thickening following balloon catheter injury has been studied in animals as a model for arterial restenosis that occurs in human patients following balloon angioplasty. Clowes, Ferns, Reidy and others have shown that deendothelilization with an intraarterial catheter that dilates an artery injures the innermost layers of medial smooth muscle and may even kill some of the innermost cells. [Schwartz, S.M., Human Pathology 18: 240 (1987); Fingerle, J., Ateriosclerosis 10: 1082 (1990)] Injury is followed by a proliferation of the medial smooth muscle cells, after which many of them migrate into the intima through fenestrae in the internal elastic lamina and proliferate to form a neointimal lesion.

Vascular stenosis can be detected and evaluated using angiographic or sonographic imaging techniques [Evans, R.G., JAMA 265: 2382 (1991)] and is often treated by percutaneous transluminal coronary angioplasty (balloon catheterization). Within a few months following angioplasty, however, the blood flow is reduced in approximately 30-40 percent of these patients as a result of restenosis caused by a response to mechanical vascular injury suffered during the angioplasty procedure, as described above. [Pepine, C., Circulation 81: 1753 (1990); Hardoff, R., J. Am. Coll. Cardiol. 15 1486 (1990)].

In an attempt to prevent restenosis or reduce intimal smooth muscle cell proliferation following angioplasty, numerous pharmaceutical agents have been employed clinically, concurrent with or following angioplasty. Most pharmaceutical agents employed in an attempt to prevent or reduce the extent of restenosis have been unsuccessful. The following list identifies several of the agents for which favorable clinical results have been reported: lovastatin [Sahni, R., Circulation 80 (Suppl.) 65 (1989); Gellman, J., J. Am. Coll. Cardiol. 17: 251 (1991)]; thromboxane $A_2$ synthetase inhibitors such as DP- 1904 [Yabe, Y., Circulation 80 (Suppl.) 260 ( 1989)]; eicosapentanoic acid [Nye, E., Aust. N.Z. J. Med. 20: 549 (1990)]; ciprostene (a prostacyclin analog) [Demke, D., Brit. J. Haematol 76 (Suppl.): 20 (1990); Darius, H., Eur. Heart J. 12 (Suppl.): 26 (1991)]; trapidil (a platelet derived growth factor) [Okamoto, S., Circulation 82 (Suppl.): 428 (1990)]; angiotensin converting enzyme inhibitors [Gottlieb, N., J. Am. Coll. Cardiol. 17 (Suppl. A): 181A (1991)]; and low molecular weight heparin [de Vries, C., Eur. Heart J. 12 (Suppl.): 386 (1991)].

In an attempt to develop better agents for preventing or reducing smooth muscle proliferation and intimal thickening, the use of balloon catheter induced arterial injury in a variety of mammals has been developed as a standard model of vascular injury that will lead to intimal thickening and eventual vascular narrowing. [Chevru, A., Surg. Gynecol. Obstet. 171: 443 (1990); Fishman, J., Lab. Invest. 32: 339 (1975); Haudenschild, C., Lab. Invest. 41: 407 (1979); Clowes, A.W., Lab. Invest. 49: 208 (1983); Clowes, A.W., J. Cardiovas. Pharm. 14: S12 (1989); and Ferns, G.A., Science 253: 1129 (1991)]. Many compounds have been evaluated in this standard animal model. The immunosuppressive agent cyclosporin A has been evaluated and has produced conflicting results. Jonasson reported that cyclosporin A caused an inhibition of the intimal proliferative lesion following arterial balloon catheterization in vivo, but did not inhibit smooth muscle cell proliferation in vitro. [Jonasson, L., Proc.Natl. Acad. Sci. 85: 2303 (1988)]. Ferns, however reposed that when de-endothelilized rabbits were treated with cyclosporin A, no significant reduction of intimal proliferation was observed in vivo. Additionally, intimal accumulations of foamy macrophages, together with a number of vacuolated smooth muscle cells in the region adjacent to the internal elastic lamina were observed, indicating that cyclosporin A may modify and enhance lesions that form at the sites of arterial injury. [Ferns, G.A., Circulation 80 (Supp): 184 (1989); Ferns, G., Am. J. Path. 137: 403 (1990)].

Rapamycin, a macrocyclic triene antibiotic produced by Streptomyces hygroscopicus [U.S. Patent 3,929,992] has been shown to prevent the formation of humoral (IgE-like) antibodies in response to an albumin allergic challenge [Martel, R., Can. J. Physiol. Pharm. 55: 48 (1977)], inhibit murine T-cell activation [Staruch, M., FASEB 3: 3411 (1989)], prolong survival time of organ grafts in histoincompatible rodents [Morris, R., Med. Sci. Res. 17: 877 (1989)], and inhibit transplantation rejection in mammals [Calne, R., European Patent Application 401,747]. Rapamycin blocks calcium-dependent, calcium-independent, cytokine-independent and constitutive T and B cell division at the G1-S interface. Rapamycin inhibits gamma-interferon production induced by I1-1 and also inhibits the gamma-interferon induced expression of membrane antigen. [Morris, R.E., Transplantation Rev. 6: 39 (1992)].

DESCRIPTION OF THE INVENTION

This invention relates to the use of rapamycin in the manufacture of a medicament for use in preventing or treating hyperproliferative vascular disease in a mammal in which the hyperproliferative disease is caused by a mechanically mediated vascular injury. The administration may be by one or more of the following routes: orally, parenterally, intravascularly, intranasally, intrabronchially, transdermally, rectally, or via a vascular stent impregnated with rapamycin.

As such, rapamycin is useful in preventing or treating intimal smooth muscle cell hyperplasia, restenosis, and vascular occlusion in a mammal, particularly following mechanically mediated vascular injury. Mechanically mediated vascular injury includes, but is not limited to vascular injury caused by catheterization procedures or vascular scraping procedures such as percutaneous transluminal coronary angioplasty; vascular surgery; transplantation surgery; laser treatment; and other invasive procedures which disrupt the integrity of the vascular intima or endothelium.

Preventing includes the prophylactic prevention of hyperproliferative vascular disease in which the hyperproliferative disease is caused or is to be caused by a mechanically mediated vascular injury in a susceptible mammal and treating includes arresting the development, and retarding the progression of hyperproliferative vascular disease in which the hyperproliferative disease is caused or is to be caused by a mechanically mediated vascular injury in a susceptible mammal.

This invention also provides a method of using a combination of rapamycin and mycophenolic acid for the same utilities described above. Mycophenolic acid, an antiproliferative antimetabolite, inhibits inosine monophosphate dehydrogenase and guanosine monophosphate synthetase, enzymes in the de novo purine biosynthetic pathway. This results in an inhibition of DNA synthesis which causes an accumulation of cells at the G1-S interface. Other combinations containing rapamycin that are useful for preventing or treating hyperproliferative vascular disease will be apparent to one skilled in the art. These include, but are not limited to, using rapamycin in combination with other antiproliferative antimetabolites.

Accordingly this invention provides a product containing rapamycin and an antiproliferative antimetabolite such as mycophenolic acid as a combined preparation for simultaneous, separate or sequential use in preventing or treating hyperproliferative vascular disease. In a further respect this invention provides a pharmaceutical composition comprising rapamycin, an antiproliferative antimetabolite such as mycophenolic acid and a pharmaceutically acceptable carrier.

The effect of rapamycin on hyperproliferative vascular disease was established in an in vitro and an in vivo standard pharmacological test procedure that emulates the hyperproliferative effects observed in mammals that are undergoing intimal smooth muscle proliferation and are therefore developing restenosis. Cycloporin A was also evaluated in these test procedures for the purpose of comparison. The combination of rapamycin and mycophenolic acid was evaluated in the in vivo test procedure. The procedures and the results obtained are described below

Rapamycin and cyclosporin A were evaluated in an in vitro standard pharmacological test procedure which emulates the intimal smooth muscle cell proliferation observed following vascular injury. Results were obtained by measuring DNA and protein synthesis in rat smooth muscle cells that have been stimulated with a growth factor such as fetal calf serum or a hypertrophic mitogen, such as angiotensin II. The following briefly describes the procedure that was used. Rat smooth muscle cells were maintained in a 1:1 mixture of defined Eagle's medium (DEM) and Ham's F12 medium with 10% fetal calf serum, penicillin (100 U/mL), streptomycin (100 mg/mL) and 25 mL Hepes at pH 7.4. Cells were incubated at 37°C in a humidified atmosphere of 5% $CO_2$ with media changes every 2-3 days. Each compound tested was diluted with an appropriate vehicle to obtain a 1 mM stock solution. Ethanol was used as the vehicle for rapamycin and 20% tween 80 in ethanol was the vehicle for cyclosporin A. Test concentrations of drug were obtained by diluting appropriate concentrations of stock solution with serum free media. The smooth muscle cell culture was maintained in a defined serum free media containing 1:1 DEM and Ham's F12 medium, insulin ($5 \times 10^{-7}$ M), transferrin ($5 \mu g/mL$), and ascorbate (0.2 mM) for 72 hours before testing in a multi-well plate. After the 72 hour period, an appropriate quantity of stock solution containing either rapamycin or cyclosporin A was added to the smooth muscle cell culture and media mixture. After a 24 hours the appropriate growth factor was added. For the measurement of DNA synthesis, $^3$H-thymidine was added at 12 hours after the growth factor was added, and the cells were harvested at 36 hours. For the measurement of protein synthesis, $^3$H-leucine was added at 14 hours after the growth factor was added, and the cells were harvested at 18 hours. The amount of incorporated radioactive label was measured on a scintillation counter.

The following table shows the results obtained for rapamycin on DNA and protein synthesis in smooth muscle cells that were stimulated with 10% fetal calf serum, as measured by incorporation of tritiated thymidine or leucine into smooth muscle cells. The amount of yitiated label incorporated by the smooth muscle cells that were treated with media only was normalized to 100%, and the results for cells treated with fetal calf serum or fetal calf serum plus the test compound are expressed as a percent comparison with the cells treated with media only.

| EFFECT OF RAPAMYCIN ON DNA AND PROTEIN SYNTHESIS IN SMOOTH CELLS STIMULATED WITH FETAL CALF SERUM* | | |
|---|---|---|
| | $^3$H-Thymidine Incorporation (% of Media) | $^3$H-Leucine Incorporation (% of Media) |
| Media | 100% | 100% |
| FCS | 495% | 174% |
| 1000 nM RAP + FCS | 136% | 95% |
| 100 nM RAP + FCS | 172% | 91% |
| 10 nM RAP + FCS | 204% | 74% |
| 1 nM RAP + FCS | 403% | 106% |

* Abbreviations: RAP = rapamycin; Media = defined serum free media; and FCS = 10% fetal calf serum.

The following table shows the results obtained for rapamycin on protein synthesis in smooth muscle cells that were stimulated with $10^{-6}$ nM angiotensin II, as measured by incorporation of tritiated leucine into smooth muscle cells. The amount of tritiated label incorporated by the smooth muscle cells that were treated with media only were normalized to 100%, and the results for cells treated with angiotensin or angiotensin plus the test compound are expressed as a percent comparison with the cells treated with media only.

| EFFECT OF RAPAMYCIN ON PROTEIN SYNTHESIS IN SMOOTH CELLS STIMULATED WITH ANGIOTENSIN II* | |
|---|---|
| | $^3$H-Leucine Incorporation (% of Media) |
| Media | 100% |
| ANG | 159% |
| 1000 nM RAP + ANG | 53% |
| 100 nM RAP + ANG | 57% |
| 10 nM RAP + ANG | 61% |
| 1 nM RAP + ANG | 60% |

* Abbreviations: RAP = rapamycin; Media = defined serum free media; and ANG = $10^{-6}$ nM angiotensin II.

The results of the standard in vitro test procedure showed that rapamycin had a pronounced antiproliferative effect in the presence of FCS and an anti-hypertrophic effect in the presence of angiotensin II. Following vascular injury, DNA and protein synthesis of smooth muscle cells are necessary for the development of restenosis to occur. These results showed that rapamycin inhibited both DNA and protein synthesis in stimulated smooth muscle cells. An antiproliferative effect was also observed with cyclosporin A; however, at 1000 nM, cyclosproin A was cytotoxic and not merely cytostatic. At 1000 nM, cyclosporin A caused lysis of the smooth muscle cells as evidenced by the presence of lactic acid dehydrogenase in the supernatent of the cell culture. Similar toxicity to smooth muscle cells was not observed for rapamycin.

Rapamycin, rapamycin plus mycophenolic acid, and cyclosporin A were evaluated in an in vivo standard pharmacological test procedure that emulates the vascular injury suffered and restenosis that develops following percutaneous transluminal coronary angioplasty in humans. The ability of a test compound to inhibit restenosis was determined by comparing intimal thickening in mammals treated with test compound following balloon catheterization versus intimal thickening in untreated control mammals after the same test procedure. [Chevru, A., Surg. Gynecol. Obstet. 171: 443 (1990); Fishman, J., Lab. Invest. 32: 339 (1975); Haudenschild, C., Lab. Invest. 41: 407 (1979); Clowes, A.W., Lab. Invest. 49: 208 (1983); Clowes, A.W., J. Cardiovas. Pharm. 14: S12 (1989); and Ferns, G.A., Science 253: 1129 (1991)]. The following briefly describes the procedure that was used. The left carotid arteries of male Sprague-Dawley rats were injured with an inflated 2Fr balloon catheter. During a 14 day postoperative period, these rats were divided into groups and treated daily with rapamycin (1.5 mg/kg; i.p.), rapamycin plus mycophenolic acid (1.5 mg/kg; i.p. + 40 mg/kg; p.o.), or cyclosporin A (3 mg/kg; i.p.). Additionally, one group each also received rapamycin (6 mg/kg/day; i.p.) or cyclosporin A (40 mg/kg/day; i.p.) for two days postoperatively, and then received no treatment for the next 12 days. An untreated group was used an injured control to establish the amount of intimal growth in the absence of treatment. The right carotid was used as an uninjured control in all groups. After the 14-day period, the rats were sacrificed, the carotids removed. The mean areas of the intima and blood vessel wall were measured by morphometry. Results are expressed as an intima percent which can be expressed according to the following formula:

$$\frac{\text{area of intima}}{\text{area of vessel}} * 100$$

The following table shows the results that were obtained.

| EFFECT OF RAPAMYCIN ON INTIMAL THICKENING IN INJURED CAROTID ARTERIES* | |
|---|---|
| Test Group | Intima Percent ± S.E. |
| Uninjured Control | 0.00 ± 0.00 |
| Untreated Injured Control | 33.3 ± 19.66 |
| RAP (1.5 mg/kg - 14 days) | 6.78 ± 4.69 |
| RAP (6 mg/kg - 2 days) | 16.56 ± 6.22 |
| RAP + MPA (14 days) | 1.6 ± 3.5 |
| CsA (3 mg/kg - 14 days) | 26.46 ± 27.42 |
| CsA (40 mg/kg - 2 days) | 31.14 ± 20.66 |

\* Abbreviations RAP = rapamycin; MPA = mycophenolic acid; and CsA = cyclosporin A.

These results show that treatment with rapamycin (1.5 mg/kg for 14 days) resulted in an 80% decrease in the mean percentage intimal thickening compared with the untreated injured control group. Similarly, treatment with the combination of rapamycin and mycophenolic acid produced almost a complete inhibition of intimal thickening (95% reduction in intimal thickening compared with untreated injured control). Cyclosporin A failed to produce any meaningful reduction in intimal thickening.

The results of the in vitro and in vivo standard test procedures demonstrate that rapamycin and rapamycin in combination with mycophenolic acid are useful in preventing or treating hyperproliferative vascular disease. Specifically, rapamycin is useful in preventing or treating intimal smooth muscle cell hyperplasia, restenosis, and vascular occlusion in a mammal, particularly following either biologically or mechanically mediated vascular injury, or under conditions that would predispose a mammal to suffering such a vascular injury.

Rapamycin was also evaluated in a modification of the in vivo test procedure described above. In the modified test procedure, treatment with rapamycin was stopped on day 14, as above, but the animals were not sacrificed immediately. Intimal thickening was observed when the animals were sacrificed 1, 2, or 4 weeks after treatment had been stopped. Microscopic analysis showed that endothelium regeneration had not occurred during the two week treatment period. Following cessation of treatment with rapamycin intimal proliferation, that was previously suppressed, was able to occur. These results are consistent with the results shown in the table above, in which treatment for 2 days with rapamycin followed by 12 days of no treatment inhibited intimal thickening to a lesser degree than treatment with rapamycin for 14 days. These results are expected, as in the absence on an integral endothelial layer, the intimal smooth muscle cells will proliferate. It has been shown that intimal smooth muscle cell growth does not have an inhibitory effect on normal endothelial regeneration, and that intimal smooth muscle cell proliferation ceases when the endothelial layer is established. [Reidy, M., Lab. invest. 59: 36 (1988); Chevru, A., Surg. Gynecol. Obstet. 171: 443 (1990); Fishman, J., Lab. Invest. 32: 339 (1975); Haudenschild, C., Lab. Invest. 41: 407 (1979)]. As such, treatment with rapamycin or rapamycin in combination with mycophenolic acid should be employed until endothelial healing has occurred.

When rapamycin is employed alone or in combination with mycophenolic acid in the prevention or treatment of hyperproliferative vascular disease, it can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dexyin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellent.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

Rapamycin, alone or in combination with mycophenolic acid, may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. Rapamycin, alone or in combination with mycophenolic acid, may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

Rapamycin, alone or in combination with mycophenolic acid can be administered intravascularly or via a vascular stent impregnated with rapamycin, alone or in combination with mycophenolic acid, during balloon catheterization to provide localized effects immediately following injury.

Rapamycin, alone or in combination with mycophenolic acid, may be administered topically as a solution, cream, or lotion by formulation with pharmaceutically acceptable vehicles containing 0.1 - 5 percent, preferably 2%, of active compound.

The dosage requirements vary with the particular compositions employed, the route of administration, the severity of the symptoms presented and the particular subject being treated. Based on the results obtained in the standard pharmacological test procedures, projected daily intravenous dosages of rapamycin, when administered as the sole active compound, would be 0.001 - 25 mg/kg, preferably between 0.005 - 5 mg/kg, and more preferably between 0.01 - 0.5 mg/kg. Projected daily oral dosages of rapamycin, when administered as the sole active compound or in combination with mycophenolic acid, would be 0.005 - 50 mg/kg, preferably between 0.01 - 25 mg/kg, and more preferably between 0.05 - 10 mg/kg. Projected daily intravenous dosages of mycophenolic acid, when used in combination with rapamycin, would be 0.5 - 25 mg/kg and preferrably between 5 - 25 mg/kg. Projected daily oral dosages of mycophenolic acid, when used in combination with rapamycin, would be 1 - 75 mg/kg and preferrably between 10 - 50 mg/kg.

Treatment will generally be initiated with small dosages less than the optimum dose of the compound. Thereafter the dosage is increased until the optimum effect under the circumstances is reached; precise dosages for oral, parenteral, intravascular, intranasal, intrabronchial, transdermal, or rectal administration will be determined by the administering physician based on experience with the individual subject treated. In general, rapamycin is most desirably administered at a concentration that will generally afford effective results without causing any harmful or deleterious side effects, and can be administered either as a single unit dose, or if desired, the dosage may be divided into convenient subunits administered at suitable times throughout the day.

**Claims**

1. Use of rapamycin in the manufacture of a medicament for use in preventing or treating hyperproliferative vascular disease in a mammal in which the hyperproliferative disease is caused or is to be caused by a mechanically mediated vascular injury.

2. Use as claimed in claim 1 in which the medicament is adapted for administration orally, parenterally, intravascularly, intranasally, intrabronchially, transdermally, rectally, or via a vascular stent impregnated with rapamycin.

3. Use as claimed in claim 1 or claim 2 in which the medicament comprises mycophenolic acid for simultaneous, separate or sequential administration.

**4.** A use according to any one of claims 1 to 3 wherein the hyperproliferative vascular disease is selected from intimal smooth muscle cell hyperplasia, restenosis and vascular occlusion.

**5.** A use according to any one of claims 1 to 3 wherein the hyperproliferative vascular disease is restenosis.

**6.** A use according to any one of claims 1 to 3 wherein the rapamycin is to be administered concurrent with and/or subsequent to said mammal undergoing a percutaneous transluminal coronary angioplasty procedure.

**7.** A use according to claim 4 wherein the mechanically mediated vascular injury is selected from vascular catherization, vascular scraping, percutaneous transluminal coronary angioplasty, vascular surgery and laser treatment.

**8.** Product containing rapamycin and mycophenolic acid as a combined preparation for simultaneous, separate or sequential use in preventing or treating hyperproliferative vascular disease.

**9.** A product according to claim 8 wherein the hyperproliferative vascular disease is selected from intimal smooth muscle cell hyperplasia, restenosis and vascular occlusion.

**10.** A product according to claim 8 or claim 9 wherein the hyperproliferative vascular disease is restenosis.

**11.** A product according to claim 9 in which the hyperproliferative vascular disease is caused or is to be caused by a mechanically mediated vascular injury selected from vascular catherization, vascular scraping, percutaneous transluminal coronary angioplasty, vascular surgery and laser treatment.

**12.** A pharmaceutical composition comprising rapamycin, mycophenolic acid and a pharmaceutically acceptable carrier.

**Patentansprüche**

**1.** Verwendung von Rapamycin bei der Herstellung eines Medikaments zur Verwendung zur Verhinderung oder Behandlung einer hyperproliferativen Gefäßkrankheit bei einem Säuger, wobei die hyperproliferative Krankheit durch eine mechanisch vermittelte Gefäßverletzung verursacht ist oder verursacht werden kann.

**2.** Verwendung nach Anspruch 1, wobei das Medikament zur Verabreichung auf oralem, parenteralem, intravaskulärem, intranasalem, intrabronchialem, transdermalem, rektalem Weg oder über einen mit Rapamycin getränkten Gefäßspanner ("vascular stent") angepasst ist.

**3.** Verwendung nach Anspruch 1 oder 2, wobei das Medikament Mycophenolsäure zur gleichzeitigen, separaten oder sequenziellen Verabreichung umfasst.

**4.** Verwendung nach einem der Ansprüche 1 bis 3, wobei die hyperproliferative Gefäßkrankheit ausgewählt ist aus Hyperplasie der glatten Muskelzellen der Intima, Restenose und Gefäßverschluss.

**5.** Verwendung nach einem der Ansprüche 1 bis 3, wobei die hyperproliferative Gefäßkrankheit Restenose ist.

**6.** Verwendung nach einem der Ansprüche 1 bis 3, wobei das Rapamycin gleichzeitig mit einem und/oder nachfolgend auf einen perkutanen, translumenalen Koronarangioplastie-Vorgang, dem der Säuger unterzogen wird, verabreicht wird.

**7.** Verwendung nach Anspruch 4, wobei die mechanisch vermittelte Gefäßverletzung ausgewählt ist aus Gefäßkatheterisierung, Gefäßabschabung, perkutaner translumenaler Koronarangioplastie, Gefäßchirurgie und Laserbehandlung.

**8.** Produkt, welches Rapamycin und Mycophenolsäure als kombiniertes Präparat enthält, zur gleichzeitigen, separaten oder sequenziellen Verwendung zur Verhinderung oder Behandlung einer hyperproliferativen Gefäßkrankheit.

**9.** Produkt nach Anspruch 8, wobei die hyperproliferative Gefäßkrankheit ausgewählt ist aus Hyperplasie der glatten Muskelzellen der Intima, Restenose und Gefäßverschluss.

**10.** Produkt nach Anspruch 8 oder 9, wobei die hyperproliferative Gefäßkrankheit Restenose ist.

**11.** Produkt nach Anspruch 9, wobei die hyperproliferative Gefäßkrankheit durch eine mechanisch vermittelte Gefäßverletzung ausgewählt aus Gefäßkatheterisierung, Gefäßabschabung, perkutaner translumenaler Koronarangioplastie, Gefäßchrirugie und Laserbehandlung bewirkt ist oder bewirkt werden kann.

**12.** Pharmazeutische Zusammensetzung, welche Rapamycin, Mycophenolsäure und einen pharmazeutisch akzeptablen Träger umfasst.

**Revendications**

**1.** Utilisation de la rapamycine dans la fabrication d'un médicament pour l'utilisation dans la prévention ou le traitement de la maladie vasculaire proliférative chez un mammifère dans laquelle la maladie vasculaire proliférative est provoquée ou doit être provoquée par une lésion vasculaire d'origine mécanique.

**2.** Utilisation selon la revendication 1 dans laquelle le médicament est adapté pour l'administration orale, parentérale, intravasculaire, intranasale, intrabronchique, transdermique, rectale ou via un extenseur vasculaire imprégné avec la rapamycine.

**3.** Utilisation selon la revendication 1 ou la revendication 2 dans laquelle le médicament comprend l'acide mycophénolique pour l'administration simultanée, séparée ou séquentielle.

**4.** Utilisation selon l'une quelconque des revendications 1 à 3 dans laquelle la maladie vasculaire proliférative est choisie parmi l'hyperplasie des cellules du muscle lisse de l'intima, la resténose et l'occlusion vasculaire.

**5.** Utilisation selon l'une quelconque des revendications 1 à 3 dans laquelle la maladie vasculaire proliférative est la resténose.

**6.** Utilisation selon l'une quelconque des revendications 1 à 3 dans laquelle la rapamycine doit être administrée de manière concurrente à et/ou subséquente audit mammifère subissant une procédure d'angioplastie coronaire transluminale percutanée.

**7.** Utilisation selon la revendication 4 dans laquelle la lésion vasculaire d'origine mécanique est choisie parmi la cathétérisation vasculaire, le raclage vasculaire, l'angioplastie coronaire transluminale percutanée, la chirurgie vasculaire et le traitement au laser.

**8.** Produit contenant la rapamycine et l'acide mycophénolique comme préparation combinée pour l'utilisation simultanée, séparée ou séquentielle dans la prévention ou le traitement de la maladie vasculaire proliférative.

**9.** Produit selon la revendication 8 dans lequel la maladie vasculaire proliférative est choisie parmi l'hyperplasie des cellules du muscle lisse de l'intima, la resténose et l'occlusion vasculaire.

**10.** Produit selon la revendication 8 ou la revendication 9 dans lequel la maladie vasculaire proliférative est la resténose.

**11.** Produit selon la revendication 9 dans lequel la maladie vasculaire proliférative est provoquée ou doit être provoquée par une lésion vasculaire d'origine mécanique choisie parmi la cathétérisation vasculaire, le raclage vasculaire, l'angioplastie coronaire transluminale percutanée, la chirurgie vasculaire et le traitement au laser.

**12.** Composition pharmaceutique comprenant de la rapamycine, de l'acide mycophénolique et un support pharmaceutiquement acceptable.